# EUROPEAN PATENT APPLICATION

(11) **EP 1 317 912 A1**
(43) Date of publication of application: **11.06.2003**
(21) Application number: 02258157.3
(22) Date of filing: 27.11.2002
(51) Int. Cl.: A61F 5/01

(54) **An orthotic strut component**

(30) Priority: 07.12.2001 GB 0129398
(71) Applicant: CHAS. A. BLATCHFORD & SONS LIMITED, Basingstoke Hampshire RG22 4AH (GB)
(72) Inventor: Evans, Andrew J. S., Boundstone, Farnham, Surrey GU10 4DT (GB); Kemp, Martin, Ash, Nr. Aldershot, Hampshire GU12 5AD (GB)
(74) Representative: Blatchford, William Michael

(57) **Abstract**

A strut component (12) for an orthosis, especially a knee orthosis, takes the form of a deformed ductile metallic tube (12A) containing a core (12B) of uncured plastics and fibre composite material. The tube has a cross-section which is much longer than its width and with parallel sides, and the composite core is a close-fit within the tube, the internal cross-sectional area of the tube being no more than *Kc*^{*2*} where c is the internal circumference of the tube and *k* is a number less than or equal to 0.1. When the orthosis is constructed, the strut (12) is shaped to suit the limb to be supported and heated to cure the composite core (12B).

## Description

This invention relates to an orthotic strut component for an orthosis, particularly for a lower limb orthosis, the strut comprising the combination of a metallic tube and an internal core of plastics and fibre composite material.

Generally, a conventional knee orthosis comprises a load-bearing support structure having upper and lower components joined together by a knee joint and shaped to extend alongside the patient's limb on the lateral side, or on both the lateral side and the medial side. The upper and lower components may be attached to the limb by straps or cuffs encircling the thigh and shin respectively.

At least the lower component usually takes the form of a steel or aluminium strut or sidebar which may have a flat or round cross section, the strut being cut to length from material of constant cross-section, and bent to suit the shape of the patient's limb. Each strut or sidebar is, therefore, individually tailored to the patient's body shape.

As described by R.L. Nelham in Chapter 8 of "Fibre Composite Hybrid Materials", edited by N.L. Hancox and published by Applied Science Publishers Limited in 1981, experiments have been performed on fibre-plastics composite struts with the aim of replacing conventional steel and alloy struts in order to obtain a lighter orthosis and, thereby, to reduce energy consumption in ambulation. One of the described proposals is a strut in the form of aluminium alloy tubing of oval cross section filled with carbon-fibre-reinforced-plastics (CFRP) "pre-preg" which can be manually bent to the required configuration for fitting to a patient and then heated to cure the inner core of composite material. One of the described disadvantages of this proposal is that the length of the strut which can be produced is limited owing to the difficulty of pulling the pre-preg material through the alloy tube without damaging the fibres. A second proposal, consisting of a strut made from an aluminium alloy "I" beam with CFRP pre-preg laid in the channels on either side of the beam, was preferred.

According to a first aspect of the invention, an orthotic strut component for an orthosis comprises the combination of a ductile metallic tube having a cross-section of elongate shape and an internal core of substantially uncured plastics and fibre composite material which is a close fit within the tube, wherein the tube is at least 300mm in length and has an internal cross-sectional area which is no more than *Kc*^{*2*}, where c is the internal circumference of the tube and *K* is a number less than or equal to 0.1, preferably less than 0.05. The tube is preferably made of fully annealed aluminium alloy and has a wall thickness in the range of from 0.5mm to 2.0mm, preferably 0.8mm to 1.0mm. The cross-section of the tube is preferably oblong in shape, having substantially straight sides and, typically, rounded ends, the ratio of the cross-section length to its width being in the range of from 2.0 to 4.0, preferably 3.0 to 3.5. The cross- section of the strut is preferably constant over at least the major part of its length, with a thickness in the range of from 4mm to 12mm. preferably 5mm to 8mm. The cross-section length is preferably in the range of from 15mm to 40mm, and, more preferably, 21mm to 23mms.

A preferred range for the value of *K* is from 0.045 to 0.06.

The internal core of the strut component may comprise, additionally, an inner core made of a heat-activated material such as a urethane foaming agent extending as a narrow strip or string though the interior of the core material.

The invention also provides an orthosis comprising at least one strut component as described above, the core having been cured, with the component riveted to a shin component for containing the patient's shin. The riveted fixing may comprise a plurality of rivet fasteners passing through holes drilled in the shin section and through both the aluminium outer tubing and the inner core of the strut component.

According to another aspect of the invention, a method of making an orthotic strut component for an orthosis comprises providing a tube which is of circular cross-section and at least 300mm in length, which is made of a ductile metallic material and which has an internal circumference *c*, providing an elongate tow of substantially uncured plastics and fibre composite material having a material cross-sectional area of no more than *Kc*^{*2*}, where *K* is a number less than or equal to 0.1, inserting the composite tow into the tube, and deforming the tube so that is has a deformed cross-section of elongate shape with an internal cross-sectional area substantially equal to that of the tow. The deforming step may comprise laterally compressing the tube to produce a deformed cross-section which is of oblong shape. The cross section may have substantially straight parallel sides and rounded ends, the strut thickness after deformation being in the range of from 5mm to 8mm over at least the major part of its length. The composite tow is preferably made from at least one sheet of unidirectional pre-preg, the sheet being rolled on itself with the fibres running substantially parallel to the axis of rolling, to form a rod of composite material which is easily passed through the tube prior to deformation of the latter. A length of heat-activated expansion or foaming agent may be incorporated in the initial turn or turns of the rolled-up sheet of pre-preg.

An orthosis may be made by employing the above-described steps and bending the strut to suit the limb which is to be supported, if necessary cutting it to length, and then heating the strut to cure the plastics and fibre composite, yielding a strut which is substantially free of voids within the tube. Elimination of voids is enhanced by incorporating the foaming agent which expands when the strut is heated. The cutting step is preferably performed before curing, may be left until after curing.

By arranging for the cross-section of the composite material forming the core (which may include a length of heat-activated expansion material) to be substantially less in area than the cross-sectional area of the tube prior to deformation, and then deforming the tube so that its internal cross-sectional area matches that of the composite (and foaming agent where present), the operation of inserting the composite core into the tube is facilitated to the extent that substantially no damage to the fibres of the composite occurs. The resulting strut, when cured, is significantly lighter than a strut of similar strength made exclusively of steel or an aluminium alloy.

The invention will now be described by way of example with reference to the drawings in which:-
Figure 1 is a side view of a lower limb orthosis including a strut component in accordance with the invention;
Figures 2A and 2B are respectively a perspective view and a side view of the strut component prior to assembly in the orthosis of Figure 1;
Figure 3 is a cross-section of a metallic tube and a fibre-reinforced plastics core for forming the strut component;
Figure 4 is a cross-section of the finished strut component; and
Figure 5 is a cross-section of a metallic tube and a fibre-reinforced plastics core for forming an alternative strut component.

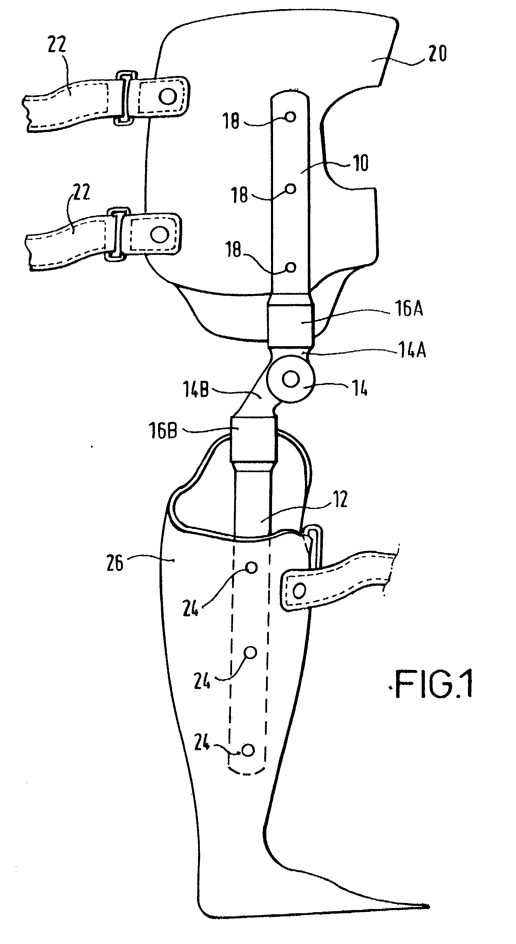

Referring to Figure 1, a lower limb orthosis has a load-bearing support structure comprising an upper strut component 10, a lower strut component 12, the strut components being connected together by a uniaxial knee joint 14. The knee joint 14 has upper and lower arms 14A, 14B carrying strut sockets 16A, 16B at their ends. These strut sockets receive the strut components 10 and 12.

The upper strut component 10 is attached by fasteners 18 to a polypropylene thigh cuff 20 having tightening straps 22. This cuff 20 is preferably custom-moulded to suit the thigh of the patient. In this case, the strut 10 is riveted to the outside of the cuff.

The lower strut component 12 is attached by fasteners 24 to a plastic shin section 26 which is shaped to extend from just below the knee to the sole of the patient's foot. The shin section 26, like the thigh cuff 20, is custom-moulded from polypropylene sheet material, the moulded shape matching that of the patient's shin and calf. To allow fitting and removal, the shin section 26 has an anterior split, bridged by a tightening strap 28. In this case, the lower strut component 12 is seated inside the shin section 26 in a longitudinal recess which is formed by placing the lower strut component 12 against a positive impression of the patient's shin and calf and then moulding the shin section over the combination of the two.

The fasteners which fasten the strut components 10, 12 to the thigh cuff 20 and shin section 26 are rivets 18, 24 which pass through holes drilled through both the struts and the cuff and shin section.

In this example, the orthosis has a lateral side support structure only, comprising the strut components 10, 12 and the interconnecting knee joint 14. It is possible to add a similar support structure on the medial side.

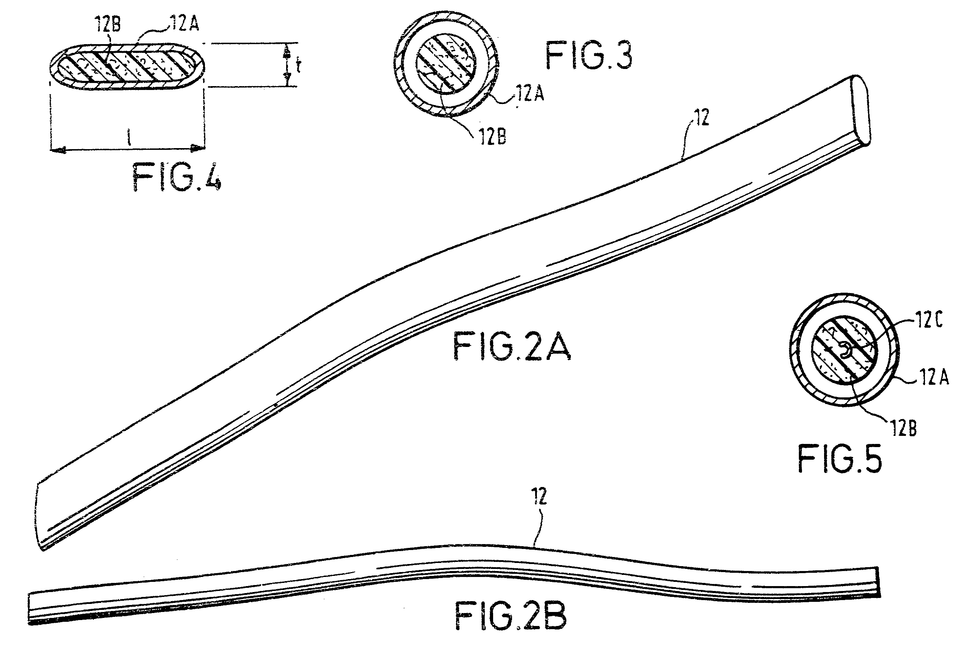

In accordance with the invention, at least the lower strut component 12 comprises the combination of a deformed aluminium alloy tube with a carbon-fibre-reinforced-plastics core. Strut component 12 is of oblong cross-section and, as shown in the perspective and side views of Figures 2A and 2B, is curved insofar as the major surfaces of the strut are non-planar to match the shape of the patient's limb. As shown in Figures 2A and 2B, bending of the strut component takes place before it is drilled to accept rivets 24 (see Figure 1).

The manufacture of the strut component and its incorporation in the orthosis described above will now be described.

Referring to Figure 3, the manufacturing process commences with a thin-walled aluminium alloy tube 12A of circular cross-section and with an outside diameter in the range of from 13mm to 20mm and a length of 300mm to 450mm. Typically, the wall thickness is in the range of from 0.8mm to 1.0mm. In a particularly preferred embodiment the tube has an outside diameter of 15.9mm and a wall thickness of 0.9mm. The tube is fully annealed to minimise resilience and, in particular, to minimise recovery spring-back after subsequent deformation. A fibre and resin composite core 12B is prepared by rolling a sheet of unidirectional carbon fibre pre-preg to form a rod in which the fibres run longitudinally. The sheet is tightly rolled to minimise voids and the sheet is dimensioned such that the cross-section of the rolled rod is substantially less than the cross-sectional area of the tube interior, as shown in Figure 3, where the tube is indicated by reference numeral 12A and the rolled pre-preg core by reference numeral 12B. The sheet is also dimensioned such that the length of the rod is at least approximately the same as that of the tube. It has been found that if the cross-sectional area of the rolled rod 12B is less than 0.1*c*^{*2*}, where c is the internal circumference of the tube, the rod 12B can be dropped down the vertically-held tube 12A substantially without damage to the fibres of the rod. This is particularly so if the cross-sectional area is less than 0.05*c*^{*2*}.

With the rod 12B located such that it extends lengthwise throughout the whole of the tube 12A, the combination of the two is next placed between the jaws of a press which is then operated to deform the tube 12A, into an oblong shape as shown in Figure 4, and, consequently, to squeeze the rod 12B. The degree of deformation is such that the internal cross-sectional area of the deformed tube 12A substantially matches the cross-sectional area of the composite rod 12B. The squeezed rod 12B is now also of oblong shape, as shown in Figure 4, and is a close fit within the deformed tube 12A, inasmuch as voids between the core and the tube are substantially eliminated. In this preferred embodiment, the quantity of pre-preg material forming the rod 12B and the dimensions of the tube 12A are chosen such that a match between the cross-sectional area of the composite and that of the tube interior is obtained when the thickness of the deformed component is approximately 7mm. To achieve the oblong shape, that is, a cross-sectional shape having straight sides and rounded ends, as shown in Figure 4, the jaws of the press have planar faces, and the approaching movement of the jaws is limited by stops to prevent compression beyond the required thickness *t*. In the present embodiment, the length *l* (see Figure 4) is 22mm.

It will be appreciated that the strut component formed in this way consists of an elongate deformed tube with a close-fitting internal core of uncured pre-preg material. This component may be stored at room temperature, or, to extend its life, it may be stored in a refrigerated place, to be supplied later to an orthosis fitting location.

The orthosis illustrated in Figure 1 is built as follows.

Firstly, the strut component with its uncured core is bent to match the shape of the limb to be supported. The shape shown in Figures 2A and 2B is typical. Since bending involves relative movement of the fibres of the core, it is preferred that the component is heated as a whole to approximately 60°C prior to bending, i.e. to a temperature sufficient to reduce the viscosity of the resin part of the core without curing it. Once the required shape has been achieved, the component is cut to a required length and is then placed in an oven and heated to a temperature and for a period sufficient to cure the pre-preg material. The component can then be drilled to accept fastening rivets.

In a modified process for forming the strut component, a thin string of a foaming material may be incorporated in the centre of the pre-preg bundle forming the rod 12B, as shown by reference numeral 12C in Figure 5. This may be produced by laying a narrow strip of the foaming material adjacent the leading (i.e. inner) edge of the pre-preg sheet referred to above, running parallel to the fibres of the sheet. When the sheet is rolled, the foaming agent strip is sandwiched between the resulting layers of pre-preg material, as shown. When the strut component is subjected to the cure cycle, the foaming agent expands and has the effect of consolidating the fibres away from the neutral axis of the component and improving the bond between the core 12B and the tube 12A. The chance of unwanted voids between the core and the tube due to any recovery of the tube away from its required oblong shape after deformation is minimised.

In the above described embodiment the fibres are substantially parallel to the longitudinal axis of the strut component but, as an alternative, some advantage may be gained in having some fibres running in a different direction. For example, outer layers with fibres running at 45 degrees to the length of the strut and wrapped around an inner core of longitudinal fibres can give an increase in torsion stiffness.

Having been drilled to accept rivets, the strut component 12 is next inserted into the strut component socket 16B, with an intervening bonding material, e.g. an epoxy resin, which is then heated to bond the strut component to the knee joint.

The upper strut component 10 is similarly bonded into socket 16A to complete assembly of the load-bearing support structure of the orthosis shown in Figure 1.

In the next stage, the strut component 12 is placed against a positive impression of the patient's shin and calf, and the shin section is moulded over both so as to reproduce the shape of the patient's limb, whilst incorporating the strut component 12 in its own recess. Holes are then drilled through the shin section in registry with the holes already drilled in the strut component 12 and rivets are fitted to secure the strut component to the shin section moulding.

The thigh cuff may be formed and attached to the strut component 10 in a similar way, or, alternatively, the upper strut component 10 may be riveted to the outside the of the thigh cuff 20, as shown in Figure 1.

The invention is not restricted to use in a leg orthosis. For instance, arm and head-neck orthoses may be constructed using a strut component or components in accordance with the invention.

## Claims

1. An orthotic strut component (12) for an orthosis, comprising the combination of a ductile metallic tube (12A) having a cross-section of elongate shape and an internal core (12B) of substantially uncured plastics and fibre composite material which is a close fit within the tube, wherein the tube is at least 300mm in length and has an internal cross-sectional area which is no more than *Kc*^{*2*}, where *c* is the internal circumference of the tube and *K* is a number less than or equal to 0.1.

2. An orthotic strut component according to claim 1, **characterised in that** the tube is made of annealed aluminium alloy and has a wall thickness in the range of from 0.5mm to 2.0mm.

3. An orthotic strut component according to claim 1 or claim 2, **characterised in that** the cross-section of the tube (12A) is of an oblong shape having substantially straight sides and rounded ends, the ratio of the cross-section length (*l*) to its width (*l*) being in the range of from 2.5 to 4.0.

4. An orthotic strut component according to any preceding claim, **characterised in that** the cross-section of the strut is substantially constant over the major part of the length of the strut component, and the thickness (*t*) of the strut over the major part is in the range of from 4mm to 12mm.

5. An orthotic strut component according to claim 1 or claim 2, **characterised in that** the cross-section of the tube (12A) is constant over the major part of its length and is of an oblong shape haying straight parallel sides and rounded ends, the cross-section length (1) being in the range of from 15mm to 40mm.

6. An orthotic strut component according to any preceding claim, **characterised in that** the value of *K* is in the range of from 0.04 to 0.10.

7. An orthotic strut component according to any preceding claim, **characterised in that** the internal core comprises an inner kernel (12C) made of a heat-activated expansion agent extending lengthwise through the strut surrounded by the said plastics and fibre composite material.

8. An orthotic strut component according to claim 7, **characterised in that** the expansion agent is a foaming agent which is formed as one or more longitudinally extending strings (12C) of material such as an epoxy resin.

9. An orthosis comprising at least one strut component as claimed in any preceding claim, the core having been cured.

10. A method of making an orthotic strut component for an orthosis, comprising providing a tube (12A) which is of circular cross-section and is at least 300mm in length, which is made of a ductile metallic material and which has an internal circumference *c*, providing an elongate tow (12B) of substantially uncured plastics and fibre composite material having a material cross-sectional area of no more than *Kc*^{*2*}, where *K* is a number less than or equal to 0.10, inserting the composite tow into the tube, and deforming the tube so that is has a deformed cross-section of elongate shape with an internal cross-sectional area substantially equal to that of the tow.

11. A method according to claim 10, **characterised in that** the deforming step comprises laterally compressing the tube to produce a deformed cross-section which is of oblong shape, and has substantially straight parallel sides and rounded ends, the strut thickness (*t*) after deformation being in the range of from 4mm to 12mm over at least the major part of its length.

12. A method according to claim 10 or claim 11, **characterised in that** the tow (12B) comprises at least one rolled-up sheet of pre-impregnated fibre and plastics composite material, the fibres of the sheet being substantially unidirectional and running substantially parallel to the axis of rolling.

13. A method according to any of claims 10 to 12, **characterised in that** the tow (12B) includes an inner elongate string (12C) of heat-activated expansion agent.

14. A method of making an orthosis, comprising the method of any of claims 10 to 13, bending the strut (12) to suit the patient's body part to be supported, and heating the strut to cure the plastics and fibre composite.
